Europäisches Patentamt

European Patent Office

Office européen des brevets

(11) Publication number: **0 062 616**
**A1**

(19)

(12) # EUROPEAN PATENT APPLICATION

(21) Application number: **82830064.0**

(22) Date of filing: **18.03.82**

(51) Int. Cl.³: **G 01 N 33/86,** G 01 N 21/27

(30) Priority: **23.03.81 IT 8334881**

(43) Date of publication of application: **13.10.82**
**Bulletin 82/41**

(84) Designated Contracting States: **AT BE CH DE FR GB LI LU NL SE**

(71) Applicant: **SALUS Ricerca e Sviluppo di Crocè Dr. Francesco & C. Snc, Viale Ungheria, 71, I-33100 Udine (IT)**

(72) Inventor: **Breda, Enzo, Via Gorizia 86, I-33100 Udine (IT)**
Inventor: **Ciotti, Alfredo, P. le Chiavris, 60, I-33100 Udine (IT)**

(74) Representative: **Petraz, Gilberto, G.L.P. S.a.s. di Gilberto Petraz P.le Cavedalis 6/2, I-33100 Udine (IT)**

(54) **Method for studying blood coagulation and the aggregation of platelets in the blood, and device employed to carry out said method.**

(57) This invention concerns a method and the relative device for sudying blood coagulation and the aggregation of platelets in the blood by analysis of the optical density of a sample of blood to which a suitable reagent may possibly have been added, so as to evaluate characteristic diagnosis parameters such as the prothrombin time (TP), the partial thromboplastin time (PTT), Howell's time (TH) and the presence of fibrinogen (FB), whereby the optical density is indicated in the form of an analogue signal (34) which is temporarily ($T_3$) zeroed before the onset ($t_0$) of the process of blood coagulation and thereafter is temporarily ($T_2$) zeroed before the onset ($t_2$) of the process of formation of fibrin, and whereby at least the characteristic parameters (TP, PTT, TH and FB) are measured by counting, starting from the moment ($t_1$) of a first surpassing of the pre-settable threshold ($d_3$ or $d_4$) by the differential signal (41) and continuing until the moment ($t_4$) of a second surpassing of the pre-settable threshold ($d_3$ or $d_4$), and whereby the method is suitable at least for obtaining directly, and substantially in true time, the time of maximum speed of reaction (K), the time of the onset of the process of formation of fibrin (R) and a parameter correlated with the quantity of active fibrinogen in the process (AM), and whereby it is possible to evaluate the value of aggregation of the platelets.

1. Description of the invention entitled:

"Method for studying blood coagulation and the aggregation of platelets in the blood, and device employed to carry out said method."

in the name of SALUS Ricerca e Sviluppo di CROCE' Dr. Francesco & C. Snc at Udine

--------

This invention concerns a method for studying the phenomena of blood coagulation and aggregation of platelets in the blood and also concerns the device employed to carry out said method.

To be more exact, the invention concerns a method which makes it possible to monitor, during the course of a process of coagulation of blood, some characteristic parameters, whether instantaneous or not, concerning the formation of fibrin and the aggregation of platelets, whereby said parameters can make possible the identification of special qualities of the blood and can therefore facilitate the diagnosis of possible deficiencies and/or defects appearing during the process of coagulation of blood.

Said process of coagulation of blood can be caused and made to evolve in various ways and periods of time by using special chemical reagents for the purpose.

The subject of the invention is especially suitable for conducting analyses regarding the determination of diagnostic

*Gilberto Petraz*

periods of time such as, for instance, the prothrombin time, the partial thromboplastin time, the calcification time or Howell's time, the presence of fibrinogen, the time of the beginning of formation of fibrin, the time of maximum speed of reaction and also the maximum final amplitude of the signal of opacity or optical density, which is correlated with the quantity of fibrinogen in the blood.

Variuos types of apparatus and methods are known for studying coagulation of blood and aggregation of platelets or for evaluating the relative diagnostic parameters.

For instance, patent US 3,833,864 comprises a digital colorimeter which enables some analyses of the elements forming the blood to be performed and which makes it possible to determine the prothrombin time by making use of modular electronic circuits which can be interchanged to suit the examination to be carried out and the wavelength of the optical radiations sent out and monitored.

Said apparatus and the relative method are complex and very expensive and necessitate the employment of various kinds of optical and electronic filters to rid the signals monitored and processed of spurious variations. This is so because the method is based on the calculation of the deri‐ vate of the signal of reaction, said derivate being expressed with a curve of a graph which must not exceed determined limits of amplitude.

Patent US 3,969,079, listed in Search Report RS 64166 IT made at the EPO in relation to the subject matter of this application, envisages an apparatus with two channels able to measure at one and the same time the prothrombin time and the partial thermoplastin time of a sample of blood.

Moreover, said apparatus is suitable for conducting other tests by using one single source of light positioned between two tests tubes arranged radially and spaced apart from each

Gilberto Ferraz

00062616

other on a rotatable plate having a circular shape.

For each test tube is provided a photo-sensor means which is able to start working only after a pre-set time so as to prevent the monitoring of false transient signals.

The diagnostic times are measured automatically from the moment of introduction of the reagent until the moment when the amplitude of the variations in optical density ex-ceeds a pre-set level within a pre-set period of time.

Patent US 3,658,480 sets forth a method and apparartus based on the computation of the derivate of the reaction signal and on comparing said derivate with a comparison signal of a pre-set amplitude, the purpose being to generate a particular signal correlated with the progress of the reaction curve.

This method is unreliable, particularly in cases of anomalous reactions, since it provides a correlated signal which may be unstable and sometimes capable of being confused with the various interferences existing in the electrical circuit.

Patent US 3,905,769 concerns a device which employs a laser beam of monochromatic light of a strong intensity; said device is, therefore, very expensive and needs skilled maintenance.

A further patent, DE-OS 2.401.084 puts forward a solution which envisages the overturning of the reaction curve upside down and the substraction therefrom of a suitable, variable comparison signal of which the variations follow the variations of said curve at least partially.

When the curve which results from said subtraction reaches a zero value, a control signal is sent out to cause the diagnosis time to be measured.

However, even the solution proposed by said patent DE OS 2.401.084 is unreliable in anomalous cases, particularly. so when the true reaction curve decreases; indeed, with this solution it is no longer possible to attain to a zeroing of the curve of the difference.

Patent application FR 7504904 (publication No. 2301011). proposes the zeroing  of the signal representing the reaction at the moment of introduction of the reagent and arran¬ ges at the same time to substract therefrom the starting value.

The optical monitoring signal is made to pass through a filter before being picked up by a photo-sensor.

This solution does not enable the diagnosis times to be determined automatically.

The above cited Search Report lists three other relevant patents, namely EP-A-0010526, GB-A-2005014 and US-A-3593568, in which pertinent methods and/or devices are disclosed.

Although, improved with respect to the prior art, these methods and/or devices are based on different principles compared with the subject matter of the present invention which represents in itself another step forward in the techniques for studying blood coagulation and platelets aggregation.

With the foregoing methods and known devices the diagnosis time are usually measured from the moment when the reagent is added, whereas it has been ascertained that the true reaction begins after a certain delay following the moment of introduction of said reagent. For this reason the diagnosis times found with the known apparatus are not very accurate.

This evaluation mistake becomes still more important owing to the addition of a mistaken evaluation of the final moment of measurement of the diagnosis time, inasmuch as said final moment is usually evaluated with empirical methods.

The purpose of this invention is to obviate said shortcomings and defects with a method for studying the coagulation of blood and the aggregation of platelets in the blood
and also with a device suitable for carrying out said method.

One purpose of the invention is, therefore, to carry out
a method of studying which can be applied to any kind of
signal monitored during the course of analysis of a phenomenon
of coagulation of blood.

A further purpose is to carry out a method which can be
applied even in the presence of an anomalous progress of the
signal monitored and/or in the presence of false variations
which may be added to the content of useful information in
said signal.

The method proposed envisages, in particular, a twofold
zeroing    of the signal monitored, whereby said twofold
zeroing makes it possible, as regards the evaluation of said
signal, to eliminate any undesired variation in the response
curve.

Said twofold zerogoing also enables a double threshold
to be pre-set for the measurement of the characteristic diagnosis times.

In particular, the two pre-set thresholds have different
signs and have amplitudes, whether the same or different,
which can be very small.

In effect, with said thresholds it is equally possible
to analyse positive or negative response curves and to detect the onset of the reaction process with a very close
approximation.

Said advantages cannot be obtained with the other methods
and devices belonging to the known art. Indeed, where single
threshold methods are in use, said single threshold enables
only curves with one single sign to be studied.

Furthermore, the lack of a twofold   zeroing   in said
known methods makes it necessary to keep a high threshold value so as to avoid the interception of variations in the signal even before the measurement of the characteristic times.

Moreover, the second zeroing makes it possible to start at once the evaluation of the quantity of fibrinogen in the blood by an automatic computation of the characteristic parameter AM.

In effect, the method and relative device which are the subject of this invention visualize that the opacity or optical density of the sample of blood, which is possibly made to react with a suitable reagent, is monitored with suitable light-detector means.

They also envisage that the signal of an analogue type correspondingly sent out by said light-detector means is processed thereafter in an analogue or digital form.

Processing in an analogue form makes it possible to obtain the characteristic diagnosis times $(T_I)$ of the tests employed such as, for instance, the prothorombin time (TP), the partial thromboplastin time (PTT), Howell's time, the presence of fibrinogen, and so on.

Digital processing makes it possible to obtain some characteristic parameters such as, for instance, the time (K) of maximum speed of reaction computed from the moment of the onset of reaction, the time (R) of the onset of the process of formation of fibrin, the maximum amplitude (AM) of the signal, which is correlated with the quantity of active fibrinogen in the process, and so on.

Suitable analogue memory means cooperating with differential amplifier means enable said analogue-digital signal to be zeroed before introduction of the reagent into the sample of blood to be analysed and, thereafter, before the process of formation of fibrin begins.

By means of suitable differential amplifier means with a twofold pre-set threshold it is possible to measure the
moment of the onset of the process and the characteristic
diagnosis time ($T_I$).

With suitable deriving means and comparator means which
make use of recording memories it is possible to evaluate
the characteristic parameters K, R and AM.

Further adapter means enable the true progress of the
signal monitored to be recorded and make it possible to
evaluate the aggregation of platelets in the sample of blood
examined.

The present invention can therefore be seen to be a method
for studying the coagulation of blood and the aggregation of
platelets in the blood by analysis of the optical density
of a sample of blood to which a suitable reagent may possibly
have been added, so as to evaluate charactetistic diagnosis
parameters such as the prothrombin time (TP), the partial
thromboplastin time (PTT), Howell's time (TH) and the presence
of fibrinogen (FB), said method being characterized by the
fact that the optical density is indicated in the form of
an analogue signal which is temporarily ($T_3$) zeroed before
the beginning ($t_o$) of the process of blood coagulation and
is thereafter temporarily ($T_2$) zeroed before the beginning
($T_2$) of the process of formation of fibrin, whereby at
least the characteristic parameters (TP, PTT, TH and FB)
are measured by counting, beginning from the moment ($t_I$)
of the first surpassing of the pre-settable threshold ($d_3$ or
$d_4$) by the differential signal (41) and continuing until
the moment ($t_4$) of the second surpassing of the pre-settable
threshold ($d_3$ or $d_4$), and whereby the method is suitable
at least for obtaining directly, and substantially in true
time, the time of maximum speed of reaction (K), the time
of the start of the process of formation of fibrin (R) and
a parameter correlated with the quantity of active fibrinogen in the process (AM), and whereby it is possible to evaluate the value of the aggregation of the platelets.

The invention also shows itself in a device able to perform said method, said device being characterized by comprising:

- light-detector means cooperating with a sample of blood to which one or more reagents may possibly have been added,
- at least one circuit to evaluate characteristic times;
- and at least one analogue-digital processing circuit.

We shall describe hereinafter an embodiment of the invention which is given as a non-restrictive example, and shall make references to the attached tables, wherein:

Fig. 1 shows a working diagram of a device which actuates the invention;

Fig. 2 gives a diagrammatic side view of possible light-detector means employed to measure the opacity or optical density of the sample of blood in a test tube;

Fig. 3 gives a time graph of the signal corresponding to one kind of evolution of opacity measured at the output of the ligh-detector means;

Fig. 4 gives a time graph of the signal corresponding to the kind of evolution of opacity illustrated in Fig. 3 and measured at the output of the summation amplifier;

Fig. 5 shows a time graph of a signal corresponding to a second kind of evolution of opacity as measured at the output of the light-detector means;

Fig. 6 shows a time graph of the signal corresponding to the kind of evolution of opacity illustrated in Fig. 5 and measured at the output of the summation ampli-fier;

Fig. 7 shows a detail of the opacity curve during the process of formation of fibrin in a case which can be measured with Fig. 8;

Gilberto Petraz

Fig.8 shows with a graph the effect of the application of a virtual threshold.

The method for studying blood coagulation phenomena according to the invention envisages the monitoring of some characteristics parameters, whether instantaneous or other-wise.

The value of said parameters depends substantiallly neither on the intrinsic opacity of the sample of blood analysed, nor on the reagents used, nor on the evolution of optical density measured during the course of the phenomenon studied, but on the true kinetic evolution of the reaction.

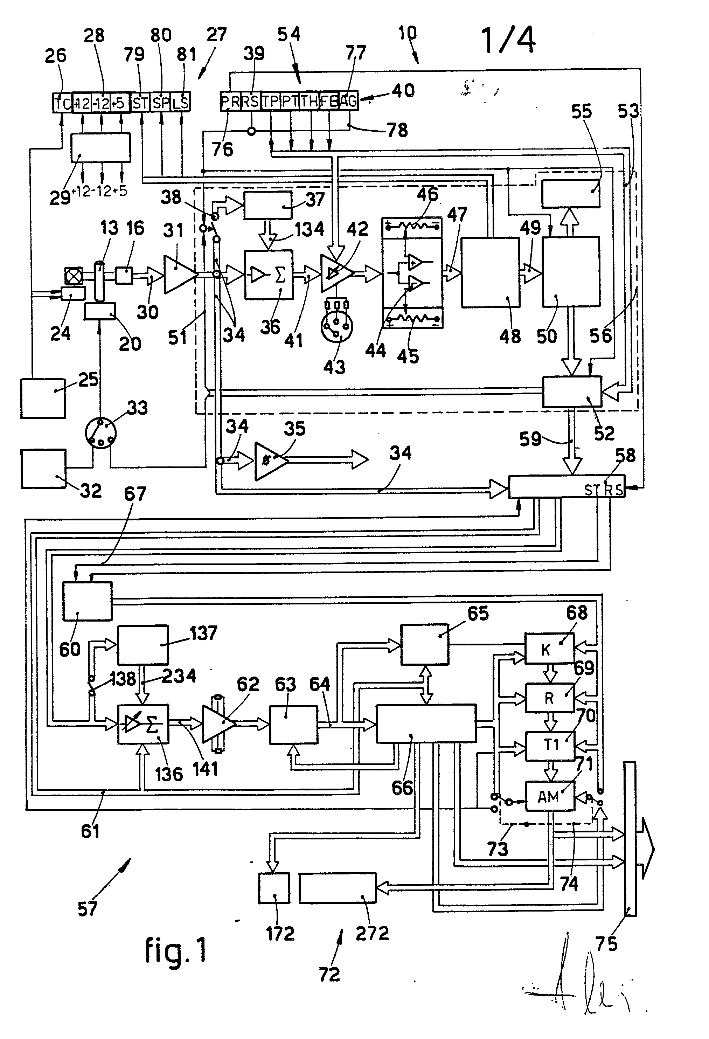

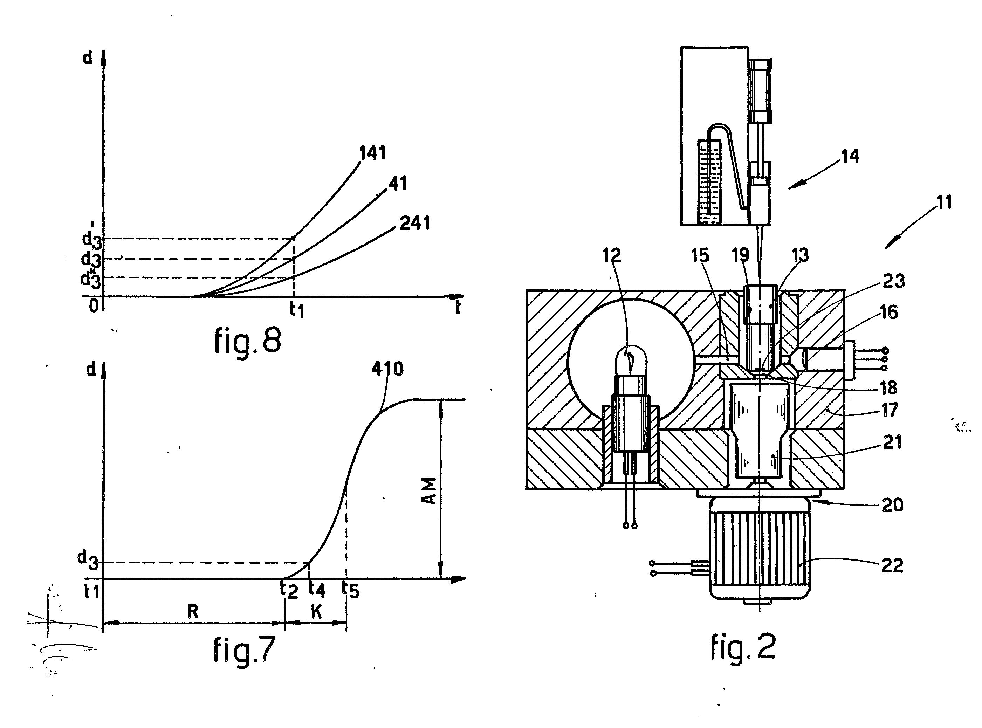

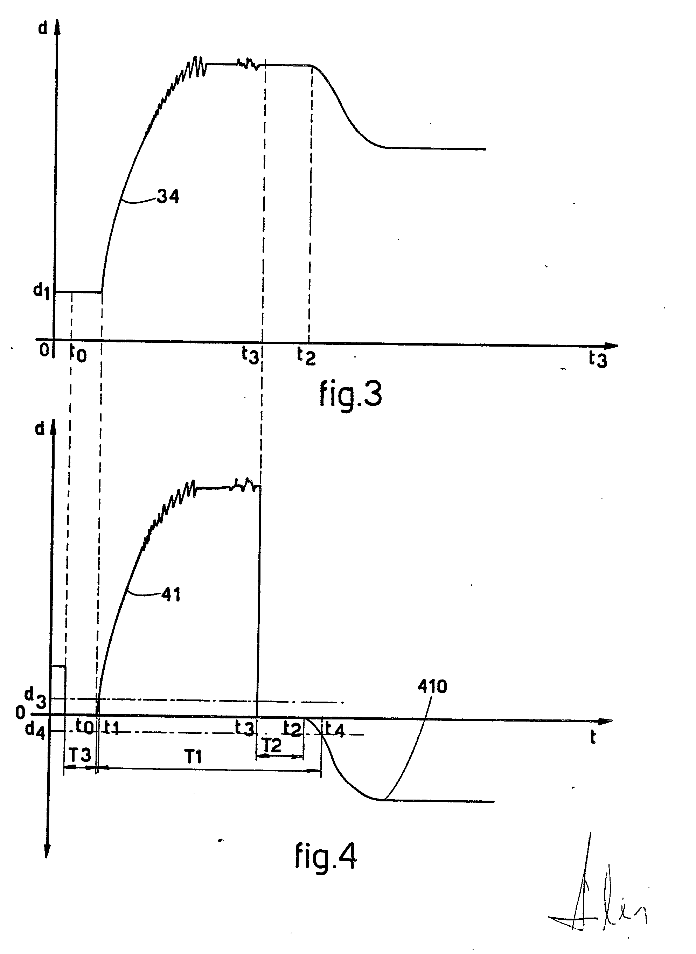

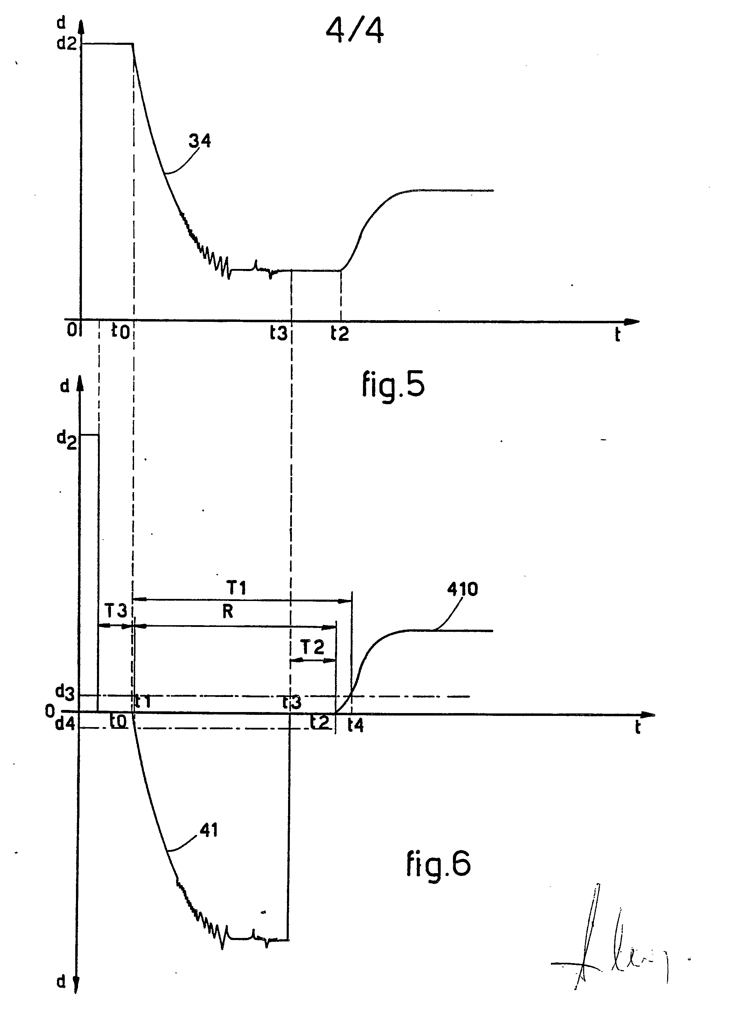

The graphs of Figs. 3 and 5 show the true evolution of optical density of a sample of blood during the blood coagulation process, wherein said evolution is monitored with suitable light-detector means 11 shown as an example in Figs. 1 and 2.

In particular, the sample of blood contained in the test tube 13 has a value of optical density which remains substantially constant until one or more reagents are introduced into said test tube 13, said reagents being capable of starting and causing the blood coagulation process.

In the examples shown, when the reagent is introduced into the test tube 13, the blood coagulation process begins and involves a variation in the optical density of the sample of blood.

In particular, said variation in optical density is accompanied at the outset by a series of transient oscillations of variable amplitude caused by the turbulence of the reagent liquids being mixed.

Said oscillations can also be possibly caused by the stirring or mechanical re-mixing to which the combination of reagents and reactive liquids may undergo at the outset.

At the end of this transient phase the value of optical density does not undergo further substantial variations until the moment when the process of formation of fibrin begins.

From that moment onwards the value of opacity varies until it reaches an absolute maximum value AM which is substantially correlated with the quantity of active fibrinogen in the process.

The reagents employed can vary, depending on the kind of examination which it is wished to carry out on the sample of blood, the purpose being to identify the qualities and anomalies of the blood being analysed.

Said reagents usually consist of chemical substances or compounds such as thromboplastin, calcium chloride, cephalin, adrenaline, etc.

With the method of the invention it is possible, in particular, to monitor some characteristic times such as, for instance, the prothrombin time (TP), the partial thromboplastin time (PTT), Howell's time (TH), the time of the onset of the reaction or of the beginning of the process of formation of fibrin, the time (K) of the maximum speed of reaction counted from the onset of said reaction, the maximum amplitude of the signal (AM), and so on.

According to the invention the following procedure is carried out to measure said parameters, bearing in mind the fact that the consequent device (10) is able to measure the optical density of which the true progress is, for instance, shown in Figs. 3 and 5 and to re-produce it in the way and amount shown in Figs. 4 and 6.

According to said figures the sample of blood plasma held in the test tube 13 has a substantially constant value of optical density until the moment when the reagent is added thereto.

In the example of Figs. 3 and 4 said constant value is shown with the reference $d_1$ and in the example of Figs. 5 and 6 with the reference $d_2$.

Said constant value is due to the fact that the sample of plasma is not disturbed by other outside reagent elements until that moment.

For a given time $T_3$ following the moment $t_0$ of introduction of the preferential reagent into the test tube, the signal, for instance the one re-produced in Figs. 4 and 6, is zeroed so as to be able to detect the first variation in signal which takes place after said moment of introduction $t_0$.

Said variation however, is detected only when one of the thresholds $d_3$ or $d_4$ of pre-settable amplitude is exceeded.

Said threshold $d_3$ and $d_4$ have different signs and can have diverse amplitudes (Figs. 4 and 6) so that it is equally possible to analyse curves having optical densities with an increasing or decreasing progress.

In this way it is not necessary to pre-arrange the analysis apparatus to suit each curve to be examined, as usually happens in the known art.

The ability to use a twofold threshold under excellent conditions is made possible owing to said zeroing carried out on said curve of optical density before beginning to read its variations.

The signal indicating the surpassing of one of the thresholds $d_3$ and $d_4$ by the curve of optical density is employed to govern the activation of a chronometer or clock or other timing means.

During the first phase of timed evolution the progress of the curve of optical density contains some oscillations of amplitude due essentially to the initial turbulence of the reagent liquids.

Said oscillations of amplitude in said curve can also be induced by a possiblle mechanical oscillation carried out to assist said process of coagulation of blood.

This part of the optical density curve does not affect the chronometric measurement in progress.

Starting from a moment $t_3$ pre-set as wished and after the moment $t_1$ of the surpassing of a threshold, a second temporary zeroing of the curve of optical density is carried out.

Said second zeroing is maintained for a period $T_2$ until just before the moment $t_2$ of the onset of the process of formation of fibrin.

Said zeroing can take place at different moments $t_3$ in relation to the kind of examination it is wished to perform and therefore to suit the type of reagent employed.

In particular, when it is wished to determine the prothrombin diagnosis (TP), the zeroing of the optical density curve is done about seven seconds from the beginning of counting (moment $t_1$).

To monitor the partial thromboplastin time (PTT), the zeroing can take place about seventeen seconds after the moment $t_1$.

To monitor Howell's time (TH), said zeroing can be done about ninety-seven seconds after $t_1$.

To proceed to monitor fibrinogen with the timed method it is possible to zero curve about four seconds after $t_1$.

Said second zeroing of the optical density curve enables the variation of said curve to be made evident, particularly at the onset of the process of formation of fibrin.

During said variation said optical density curve surpasses once again one of said threshold ($d_3$ and $d_4$) (Figs. 4 and 6).

Said surpassing is suitably decoded and makes it possible to measure the time $T_1$ which has gone by between the moment $t_1$ of the first surpassing of threshold and the moment $t_4$ of the second surpassing of threshold $d_3$ and $d_4$ (Figs. 4, 6

and 7).

Said measurement is substantially independent of what happens during the evolution of the curve from the moment $t_1$ until just before the moment $t_2$, and can be done by establishing a threshold ($d_3$ and $d_4$) which advantageously has a minimum value.

In the known art the uncertain progress of the curve of optical density does not permit the characteristic or diagnostic times to be measured very accurately. This is so owing to the methods employed, particularly when use is made of virtual threshold, which have to be kept at high values so as to avoid the reading of false variations before the onset of the reaction. The results obtained in this way are not at all accurate.

The time $T_1$ measured can assume various meanings, depending on whether it is understood to be TP or PTT or TH or depending on the kind of examination performed on the sample of blood.

When the value of the moment $T_4$ is known, it is possible to calculate the moment $t_2$ of the start of the reaction in the knowledge that $t_2$ is typically about 20% less than the value of the moment $t_4$.

In other words, the value of $t_2$ can be obtained by extrapolating to zero the curve of optical density and by taking as a base the point corresponding to the moment $t_4$ (Fig. 7).

The use of means able to calculate the derivates makes it possible to analyse, after the moment $t_4$, the slope of the curve shown in Figs. 4 and 6.

In this way it is possible to obtain both the value of maximum slope corresponding to the maximum speed of reaction and also the moment in which that event takes place.

Two further parameters which have a special diagnostic significance can be measured at once by virtue of the second zeroing of the curve of optical density. They are the maximum amplitude AM correlated with the quantity of active fibrinogen in the process and the moment when said amplitude AM, which can be ignored, is reached.

The curves of optical density 3 and 5 serve to show the process of aggregation of platelets. In particular, said aggregation is evaluated by comparing said curves of optical density, as shown in Figs. 3 and 5, with plots of a known value of the analogue signal 34.

The block working diagram of Fig. 1 shows a device 10 which enables the method for studying the phenomena of coagulation of blood and aggregation of platelets in the blood to be carried out according to the invention.

Said device 10 comprises light-detector means 11 shown in Fig. 2 and consisting of a light-emitting element 12 able to send luminous radiations through a test tube 13.

Said test tube 13 is made of optically transparent material and holds the sample of blood to be analysed. Said sample can possibly have reagent added to it and can be introduced into said test tube 13 with an appropriate injector 14.

The luminous radiations sent out by the light-emitting element 12 are guided through a through hole 15, which is made, in the example, in the support 17 of the test tube 13.

The through hole 15 has a small diameter so as to cause said radiations to assume the shape of a very narrow luminous beam.

This fact makes it possible to eliminate, or at least to lessen, the false reflections and refractions which exist, instead, when beams of light with a greater diameter are employed.

In fact, beams of light of a given diameter are unevenly reflected and refracted by the curved surface of the test tube.

Moreover, said beams of light of a known kind are more likely to be reflected by the particles in suspension in

*Gilberto Petraz*

said sample of blood.

In particular, the light-emitting element 12 can consist of a normal light bulb, as in the example shown, or of another source of white light.

The light-emitting element 12 can also consist of a light-emitting diode the luminuos radiations of which can belong to a particular band or to particular wavelengths of the optical spectrum.

It can be envisaged, for instance, that said light-emitting element send out radiations having a wavelength belonging to the infrared band.

After passing through the sample of blood to be analysed in the test tube 13, said luminous beam reaches a light-detector or optical detector 16 and is converted thereby into a suitable electrical signal of an analogue type 30 (Fig. 1).

Said light-detector 16 can be envisaged as being sensitive only to the kind of light sent out by the light-emitting element 12.

In the example of the solution shown for light-detector means 11 (Fig. 2) it is visualized that the support 17 of the test tube 13 has in its lower part an opening 18 to drain away the impurities or deposits which may possibly be present in the space 19 which holds the test tube 13.

Fig. 2 also shows the means 20 that serve to stir the sample of blood, which is possibly caused to react. In this example said means 20 consist of an electromagnet or magnet 21 and are made to rotate by motor means 22 which have, perhaps, an adjustable speed.

Said motor means 22 can be driven continuously with a supply circuit 32 (Fig. 1) or for set periods of time, or perhaps are not driven. It is possible to pre-select each of these working conditions with a selector-commutator 33.

0062616

The rotating magnetic field generated by said means 20 sets in rotation a small bar 23 made of a material sensitive to magnets and immersed in the sample of blood in the bottom of the test tube 13.

It is possible to visualize that there are as many light-detector means 11 available as there are examinations to be carried out on one and the same type of sample of blood.

In such a case said light-detector means 11 can be connected in series with one single device to process the data monitored.

The block working diagram of Fig. 1 shows thermostat means 24 which are located close to the test tube 13.

Said thermostat means 24 can consist of heating elements such as, for example, incandescent elements or heat dissipation elements.

They can also and additionally consist of heat sensors such as, for instance, resistances of a negative temperature coefficient type.

Said heat sensors are employed to monitor the ambient temperature near to said test tube 13.

A suitable temperature control circuit 25 analyses the signals coming from said heat sensors and regulates accordingly the circulation of current in the heating elements.

Any anomalies or special working conditions of the thermostat means 24 can be shown (26) on an appropriate optical indicator of functions 27.

Said optical indicator of functions 27 can show (28) the state of functioning of the power feeder 29 of the device 10.

The analogue signal 30 sent out by the light-detector 16 is correlated with the optical density or opacity of the sample of blood being examined and is amplified by a pre-amplifier 31.

The pre-amplifier 31 consists substantially of one or more operational amplifiers, with a possibly adjustable gain, connected cascade-wise.

Said amplification is performed to raise the level of the analogue signal 30 from a weak intensity to values such as will permit adequate processing thereafter.

The amplified analogue signal 34 is sent on one side to an algebraic summation circuit 36 consisting substantially of a differential amplifier and is added there algebraically to a signal 134 coming from an analogue memory.

In particular, the analogue signal 34 is fed into said analogue memory 37 by the closure of a switch 38, said closure being brought about by operating by hand an appropriate zeroing key 39 on the push-button panel 40.

When said switch 38 is opened, the amplitude of the signal 34 present in the analogue memory 37 at the moment before said opening remains stored in said memory 37.

In effect, when the switch 38 is closed, two equal analogue signals 34 reach the algebraic summation circuit 36, so that the differential signal 41 at the output of said summation circuit 36 assumes a zero value.

Instead, when the switch 38 is opened, there reach the algebraic summation circuit 36 a signal 134 of a constant value in time and an analogue signal 34 which may possibly be variable, so that the output signal 41 of said summation circuit 36 can assume other than zero.

This fact enables a differential signal 41 to be obtained which describes a new curve of optical density comprising zeroing tracts ($t_3$ and $t_2$), as shown in the example of Figs. 4 and 6.

In practice the true curve of optical density remains essentially constant until the reagent is introduced (moment $t_0$) into the test tube 13.

The first zeroing of the analogue signal 34 (Figs. 3 and 5) is then carried out by closing the switch 38.

In correspondence with the introduction of reagent (moment $t_0$) into the test tube 13 the switch 38 is opened and the differential signal 41 (Figs. 4 and 6) begins to assume values other than zero, following the progress of the analogue signal 34 (Figs. 3 and 5).

Said differential signal 41 is fed into an amplifier 42 of which the gain can be varied by acting on its polarization circuit with the push-button panel 40 and, separately, with a suitably calibrated selective commutator 43.

Said variation of the gain of the amplifier 42 enables the amplitude of the differential signal 41 to be increased (141) or reduced (241).

The outcome is that said signal 41 can surpass, in advance or with a delay, the threshold $d_3$ or $d_4$ pre-set in the threshold amplifier 44 connected immediately downstream from said amplifier 42.

In effect, an increase or decrease in the slope of the curve 41 generated by the amplifier 42 will produce respect- ively an increase $d'_3$ (Fig. 8, curve 141) or a decrease $d''_3$ (Fig. 8, curve 241) in the true threshold value $d_3$ resulting from the differential signal 41 (Fig. 8).

In other words, said variation of gain can correspond, in fact, to the establishment of a virtual threshold ($d'_3$ or $d''_3$) different from the true threshold $d_3$.

Said threshold amplifier 44 can consist of two differential amplifiers able to compare separately the differential signal 41 with reference signals which act as thresholds ($d_3$ and $d_4$) and which can be appropriately regulated with means 45 and 46.

When the differential signal 41 exceeds one of the reference signals $d_3$ or $d_4$ (said exceeding takes place at the moment $t_1$ in the example of Figs. 4 and 6), the threshold amplifier

44 sends out a DIGITAL SIGNAL 47 which enables (49) the logical circuit 48 to activate a timer or clock 50 of a digital type, for instance.

At the moment $t_3$ there take place a further closure of the switch 38 and a consequent further zeroing of the differential signal 41 (Figs. 4 or 6).

Said closure is done (51) automatically by a timer 52, which is programmed (53) beforehand by pre-selection (54),on the push-button panel 40, of the kind of examination which is wished to carry out on the sample of blood.

In effect, a moment $t_3$ having a desired value can be used, depending on whether it is wished to measure the pro-thrombin time (TP), thromboplastin time (PTT), Howell's time (TH) or the presence of fibrinogen (FB).

Said value will correspond respectively to about $t_1$ plus 7" or $t_1$ plus 17" or $t_1$ pus 97" or $t_1$ plus 4" for the examinations of TP, PTT, TH and FB.

Said additional periods (respectively 7", 17", 97" and 4") depend on the type of reagent and indicate an average value around which the desired pre-set or pre-settable value can be selected.

Said values of the moment $t_3$ ad of the time $T_2$ (Figs. 4 and 6) of closure of the switch 38 can be obtained by evaluating statistical results dependent on very extensive records of examinations.

At the end of the period of time $T_2$ the timed opening of the switch 38 takes place and makes possible the monitoring of a further surpassing of the threshold $d_3$ (or $d_4$) by a second differential signal 410.

Said second differential signal 410 is due to the variation of the optical density as an outcome of the formation of fibrin.

The measurement of said surpassing takes place at the

*Gilberto Petraz*

moment $t_4$, as shown in Figs. 4, 6 & 7.

At the moment $t_4$ the amplifier 44 sends out a signal which is utilized to operate the halting of the counting by the clock 50.

This signal sent out by the amplifier 44 also serves to make appear the relative counting time $t_1$, which has the purpose of signifying the examination TP or PTT or TH or FB, on an appropriate optical displays means 55 of an electronic display type, for instance.

The signal sent out by the amplifier 44 can possibly be converted into a signal of a digital type before being used.

The measurement of said time $t_3$ by means of the circuit 56 that evaluates characteristic times is followed by the measurement of the characteristic parameters $V_1$, R and AM by means of the specific analogue-digital processing circuit 57.

The circuit 56 consists substantially of the blocks 36-37-42-44-48-50-52-55 of Fig. 1.

In particular, there arrive at the circuit 57 (possibly by means of a group of connectors 58): - timing signals 59: the analogue signal 34: a coded signal correlated with the signal of starting-up 49 the clock 50; and the signal corresponding to the time $T_3$ measured with the circuit 56.

The timing signals 59 comprises the signal for manual zeroing, or first zeroing, pre-set with the key 39 at the beginning of the process.

The signal 59 controls the closing of the switch 38 and the zeroing of the data contained in the circuits 48 and 50.

It also (59) governs, through the logical block 60, the closing of the switch 138 located at the input of the circuit 57.

The consequent zeroing signal can possibly be suppressed by opening the logical block 60.

Owing to the action of the logical block 60 the switch .138 stays closed up to the moment $t_3$ plus $T_2$, which is just before the moment $t_2$ of the onset of the formation of fibrin (Figs. 4, 6 and 7).

From that moment $t_3$ plus $T_2$ the analogue signal under-goes the same comparison process as that which we have already described in the circuit 56 in correspondence with the logical blocks 36 and 37.

The analogue signal 34 can possibly be amplified differently in the differential amplifier 136 owing to the presence of an internal selector able to introduce diverse values of electrical grid bias resistance; said differential amplifier is enabled to do this by a sequence of signals 61 coming from the keyboard 40 through the timer 52.

The signal 141 thus generated is amplified (62) there-after and sent to an analogue-digital converter 63.

The digital signal 64 sent out is fed, on the one hand, to a deriving circuit 65 and, on the other hand, to a logic control circuit 66.

Both the circuit 65 and 66 have been pre-disposed be-forehand for processing the digital signal 64 by a sequence of control signals 61 sent out by the keyboard 40 through the timer 52 and the group of connectors 58.

The deriving circuit 65 carries out a comparison of difference between successive amplitudes of a second differential signal 40, said amplitudes being obtained with instantaneous components and said digital signal 64 being utilized.

In correspondence with the moment $T_5$ of the identification of the greater value of difference, the circuit 65 send a signal to a recording counter 68 to stop counting.

The recording counter 68 is activated beforehand by a START signal 67 sent by the circuit 56 as soon as the first

surpassing of threshold $d_3$ (or $d_4$) by the analogue signal 41 has taken place.

The characteristics time K measured in this way corresponds to the time which goes by from the moment $t_2$ until the moment when there is the maximum speed of reaction, which in Fig. 7 corresponds to $t_5$.

The logic control circuit 66 contains the circuit generating signals of clock, or of cadence, which serves for the working of the recording counters 68, 69, 70 and 71 and for the working of the analogue-digital converter 63.

Said logic control circuit 66 is suitable for halting the counting of a recording counter 69, so that the time R which has gone by from the beginning of the process $(t_1)$ until the moment $t_2$ of the onset of reaction remains recorded therein (69).

In particular, the time R can be calculated by knowing that the moment $t_2$ can be made to correspond, with a good approximation, to the moment $t_3$ plus $T_2$ of the end of the second zeroing.

In other words, the time R can be calculated by knowing that it is generally 20% less than the time $T_1$ supplied by the test.

The recording counter 69 stores the time $T_1$, whereas the recording counter 70 stores the maximum value AM of optical density corresponding with the end of the reaction process.

In particular, said maximum value AM is computed in the logic control circuit 66 by successive comparisons of the amplitudes of the digital signal 64.

The data recorded can be shown on an appropriate optical display 72 comprising a display zone 172 reserved for the code relative to the type of examination and a zone 272 reserved for the relative value recorded.

A double commutator 73 is also comprised which perhaps may make it possible to send (74) the analogue signal corresponding to the value of T  or the digital values recorded relating to the parameters R, K and AM separately to a printer unit or another outside user means through a group of connectors 75.

The command to send said data to the printer can be sent out from the keyboard 40 by operating an appropriate key 76.

Furthermore, by operating a key 77 it is possible to display or record only the signal correlated with the aggregation of platelets in the blood.

This can be done, in particular, by closing (78) the switch 38 so as to stop the processing of the signal 34 in the circuit 56 and by sending the same signal 34 to a circuit 35 which adapts impedance and amplitude, so as to enable the device 10 to be coupled to the outside recorder and/or display means.

The curves of optical density recorded and/or displayed in this way have, in this example, the true progress shown in Figs. 5 and 7.

According to the embodiment of the device 10 shown in Fig. 1 it is possible to show with the optical indicator of functions 27 the operating conditions (79) of the counter 50, of the stop (80) of said counter 50 and of the surpassing (81) of threshold $d_3$ (or $d_4$).

We have described here a preferential embodiment of this invention but variants are possible for a technician in this field. Thus the forms and sizes of the light-detector or means 11 can be changed, and it is possible to envisage that the working blocks of the device 10 shown in Fig. 1 are embodied with different electrical and electronic components while retaining the working characteristics described and shown, etc.

These and other variants are all possible for a technician in this field without departing thereby from the scope of the idea of the solution.

C L A I M S

1 - Method for studying blood coagulation and the aggregation of platelets in the blood wby analysis of the optical density of a sample of blood to which a suitable reagent may possibly have been added, so as to evaluate characteristic diagnosis' parameters such as the prothrombin time (TP), the partial thromboplastin time (PTT), Howell's time (TH) and the presence of fibrinogen (FB), said method being characterized by the fact that the optical density is indicated in the form of an analogue signal (34), which is temporarily $(T_3)$ zeroed before the onset $(t_0)$ of the process of coagulation of blood and is temporarily $(T_2)$ zeroed thereafter before the beginning $(t_2)$ of the process of formation of fibrin, whereby at least the characteristic parameters (TP, PTT, TH and FB) are measured by counting, starting from the moment $(t_1)$ of the first surpassing of the pre-settable threshold $(d_3$ or $d_4)$ by the differential signal (41) and continuing until the moment $(t_4)$ of the second surpassing of the pre-settable threshold $(d_3$ or $d_4)$, and whereby the method is suitable at least for obtaining directly, and substantially in true time, the time maximum speed of reaction (K), the time of the onset of the process of formation of fibrin (R) and a parameter correlated with the quantity of active fibrinogen in the process (AM), and whereby it is possible to evaluate the value of aggregation of platelets.

2 - Method for studying blood coagulation and the aggregation of platelets in the blood by analysis of the optical density of a sample of blood to which a suitable reagent may possibly have been added, as in claim 1, characterized by the fact that the analogue signal (34) corresponding to the variation in optical density is changed by two zeroings, which are carried out respectively before the moment $(t_0)$ of the onset of the blood coagulation process and at a moment $(t_3)$ chosen

as desired, into a first (41) differential signal and a second (410) differential signal read as an analogue value (410) and a digital value (64), whereby the second temporary zeroing $(T_2)$ begins at the moment $(t_3)$ chosen as desired and end just before the onset $(t_2)$ of the process of formation of fibrin.

3 - Method for studying blood coagulation and the aggregation of platelets in the blood by analysis of the optical density of a sample of blood to which a suitable reagent may possibly have been added, as in Claim 1 or 2, characterized by the fact that the analogue signal (34) corresponding to the variation in optical density is read either as an increasing value or as a decreasing value, whereby two pre-settable thresholds, respectively $(d_3)$ and $(d_4)$ cooperate with the differential signal (41-410).

4 - Method for studying blood coagulation and the aggregation of platelets in the blood by analysis of the optical density of a sample of blood to which a suitable reagent may possibly have been added, as in claim 1 or 2 or 3, characterized by the fact that the second differential signal (410) can be amplified.

5 - Method for studying blood coagulation and the aggregation of platelets in the blood by analysis of the optical density of a sample of blood to which a suitable reagent may possibly have been added, as in claim 1 or 2 or 3, characterized by the fact that the threshold $(d_3$ or $d_4)$ at least in cooperation with the second differential signal (410) can be varied as wished.

6 - Method for studying blood coagulation and the aggregation of platelets in the blood by analysis of the optical density of a sample of blood to which a suitable reagent may possibly have been added, as in claim 1 and in one or another of the claims thereafter up to claim 5 inclusive, characterized by

0062616

the fact that the moment $(t_2)$ of the onset of the reaction is measured.

7 - Method for studying blood coagulation and the aggregation of platelets in the blood by analysis of the optical density of a sample of blood to which a suitable reagent may possibly have been added, as in claim 1 and in one or another of the claims thereafter up to claim 5 inclusive, characterized by the fact that the moment $(t_2)$ of the onset of the reaction is obtained by subtracting a pre-settable value from the value of the moment $(t_4)$ of the second surpassing of the threshold $(d_3$ or $d_4)$ by the second differential signal (410) expressed in digital form (64).

8 - Method for studying blood coagulation and the aggregation of platelets in the blood by analysis of the optical density of a sample of blood to which a suitable reagent may possibly have been added, as in claim 1 and in one or another of the claims thereafter, characterized by the fact that the measurement, substantially in true time, of the time of the onset of the process of formation of fibrin (R) is evaluated in a digital form (64) starting from the moment $(t_1)$ of the first surpassing of the threshold $(d_3$ or $d_4)$ by the differential signal (41) and continuing until the moment $(t_2)$ of the onset of the reaction.

9 - Method for studying blood coagulation and the aggregation of platelets in the blood by analysis of the optical density of a sample of blood to which a suitable reagent may possibly have been added, as in claim 1 and in one or another of the claims thereafter, characterized by the fact that the measurement, substantially in true time, of the time of attainment of maximum speed of reaction (K) is evaluated in digital form (64), starting from the moment $(t_2)$ of the onset of the reaction and continuing until the moment $(t_5)$ of maximum speed of reaction.

10- Method for studying blood coagulation and the aggregation

of platelets in the blood by analysis of the optical density·
of a sample of blood to which a suitable reagent may possibly
have been added, as in claims 1 and 9, characterized by the ·
fact that the moment ($t_5$) of maximum speed of reaction is
obtained by comparing differences between successive amplitudes
of the second differential signal (410) obtained by means of·
instantaneous samplings, whereby a digital signal (64) is
utilized.

11- Method for studying blood coagulation and the aggregation
of platelets in the blood by analysis of the optical density·
of a sample of blood to which a suitable reagent may possibly*
have been added, as in claim 1 and in one or another of the ·
claims thereafter, characterized by the fact that the parameter
correlated with the quantity of fibrinogen in the blood (AM)·
is evaluated in a digital form (64) by means of successive
comparisons of the sample amplitudes of the second differential
signal (410) measured.

12- Method for studying blood coagulation and the aggregation
of platelets in the blood by analysis of the optical density·
of a sample of blood to which a suitable reagent may possibly
have been added, as in claim 1 and in one or another of the ·
claims thereafter, characterized by the fact that the
aggregation of platelets is evaluated by comparison with
plots of a known value of the analogue signal (34).

13- Device for studying blood coagulation and the aggregation
of platelets in the blood by analysis of the optical density·
of a sample of blood to which a suitable reagent may possibly
have been added, so as to evaluate characteristic diagnosis ·
parameters such as the prothrombin time (TP), the partial
thromboplastin time (PTT), Howell's time (TH) and the presence
of fibrinogen (FB), being suitable at least for obtaining
directly, and substantially in true time, the time of maximum
speed of reaction (K), the time of the onset of the process/.

of formation of fibrin (R) and a parameter correlated with
the quantity of active fibrinogen in the process (AM), whereby
it is possible to evaluate the value of the aggregation of
platelets, said device being characterized by carrying out
the method of the claims hereinbefore and by comprising in
mutual cooperation with light-detector means (11):

- means for the temporarily ($T_3$ and $T_2$) zeroing (36-37) of
  analogue signal (34), being operated at a first time by
  a zeroing key (39) and at a second time by a timer (52),
- adjustable (45-46) threshold amplifier (44) means,
- logical circuit means (48) linked to the threshold amplifier
  (44) means and cooperating with a time counter or clock (50),
- a circuit to evaluate characteristic times (56)
- and an analogue-digital processing circuit (57).

14- Device for studying blood coagulation and the aggregation
of platelets in the blood by analysis of the optical density
of a sample of blood to which a suitable reagent may possibly
have been added, as in claim 13, characterized by the fact
that the circuit for evaluating characteristic times (56)
comprises in coordinated cooperation at least:

- means (36-37) for the temporary ($T_3$ and $T_2$) zeroing of the
  analogue signal (34) which are operated at a first time by
  a zeroing key (39) and at a seocnd time by a timer (52),
- adjustable (45-46) threshold amplifier (44) means,
- and logical circuit means (48) linked to the threshold
  amplifier means (44) and cooperating with a time counter
  or clock (50).

15- Device for studying blood coagulation and the aggregation
of platelets in the blood by analysis of the optical density
of a sample of blood to which a suitable reagent may possibly
have been added, as in claim 13 or 14, characterized by the
fact that the analogue-digital processing circuit (57) comprises
in coordinated cooperation at least:

0062616

— 30 —          rif. sie 4-3229

.- means (136-137) for continuous zeroing until just before

. the moment ($t_2$) of the onset of the process of formation

. of fibrin,

.- analogue-digital convertor means (63),

.- deriving circuit means (65)

.- and recording counter means (68-69-70-71).

.16- Method for studying blood coagulation and the aggregation

.of platelets in the blood by analysis of the optical density.

.of a sample of blood to which a suitable reagent may possibly.

.have beed added, and device suitable for carrying out said

.method, as in one or more of the claims hereinbefore and as .

.described and shown and for the purposes allowed.

*Gilberto Piettaz*

fig.1

0062616

1/4

fig.2

fig.8

fig.7

3/4

fig.3

fig.4

4/4

fig.5

fig.6

European Patent
Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| A,D | EP-A-0 010 526  (SALUS ISTITUTO DIAGNOSTICO) *Pages 2-4,6,7; figure 1* | 1 | G 01 N  33/86 G 01 N  21/27 |
| A,D | US-A-3 969 079  (J.CATARIOUS et al.) *Column 5,6; figure 7* | 1 | |
| A,D | GB-A-2 005 014  (SANKYO CO.) *Pages 2-4; figures 4-6* | 1 | |
| A,D | US-A-3 593 568  (W.SCHMITZ et al.) *Column 2,3,12,13; figures 1,7* | 1 | |

TECHNICAL FIELDS
SEARCHED (Int. Cl. ³)

G 01 N  33/86
G 01 N  33/48
G 01 N  21/27

The present search report has been drawn up for all claims

| Place of search THE HAGUE | Date of completion of the search 24-07-1982 | Examiner BOEHM CH.E.D. |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82